# Europäisches Patentamt

(19) **European Patent Office**

Office européen des brevets

(11) Veröffentlichungsnummer : **0 285 563 B1**

## (12) EUROPÄISCHE PATENTSCHRIFT

(45) Veröffentlichungstag der Patentschrift :
**29.04.92 Patentblatt 92/18**

(21) Anmeldenummer : **88810191.2**

(22) Anmeldetag : **24.03.88**

(51) Int. Cl.⁵ : **A61K 31/565,** A61K 47/10,
A61L 15/16

(54) Transdermale therapeutische Systeme für Wirkstoffkombinationen.

(30) Priorität : **02.04.87 CH 1259/87**

(43) Veröffentlichungstag der Anmeldung :
**05.10.88 Patentblatt 88/40**

(45) Bekanntmachung des Hinweises auf die
Patenterteilung :
**29.04.92 Patentblatt 92/18**

(84) Benannte Vertragsstaaten :
**AT BE CH DE ES FR GB GR IT LI LU NL SE**

(56) Entgegenhaltungen :
**EP-A- 0 136 011
EP-A- 0 196 769**

(56) Entgegenhaltungen :
**EP-A- 0 253 607
EP-A- 0 275 716
EP-A- 0 279 977
GB-A- 2 093 694
US-A- 3 968 245**

(73) Patentinhaber : **CIBA-GEIGY AG
Klybeckstrasse 141
CH-4002 Basel (CH)**

(72) Erfinder : **Fankhauser, Peter, Dr.
Hauptstrasse 65
CH-4107 Ettingen (CH)**
Erfinder : **Schenkel, Lotte, Dr.
Nadelberg 29
CH-4051 Basel (CH)**

**Beschreibung**

Die vorliegende Erfindung betrifft ein therapeutisches System für die transdermale kombinierte Applikation von 17β-Oestradiol mit Norethisteron-17-acetat, Verfahren zur Herstellung dieses Systems und die Anwendung dieser Wirkstoffkombination in transdermalen therapeutischen Systemen zur Behandlung des klimakterischen Syndroms.

Das klimakterische Syndrom umfasst alle nach der Menopause auftretenden, durch sogenannten Oestrogenmangel bedingten Ausfallerscheinungen. Die seit langem bekannte Oestrogensubstitution ist prophylaktisch und therapeutisch bedeutsam. Hitzewallungen, Schlafstörungen und Dysphorie werden günstig beeinflusst. Wesentlich ist die Senkung der Morbidität und der Mortalität aufgrund osteoporotisch bedingter Frakturen sowie koronarer und zerebrovaskulärer Erkrankungen, siehe hierzu P.J. Keller, Schweiz. Rundschau Med. (Praxis) 75, Nr. 12 (1986), S. 328.

Bei der oralen Verabreichung von Oestrogenen, z.B. 17β-Oestradiol, ist die Resorption wegen ihrer geringen Wasserlöslichkeit nach oraler Applikation ungenügend. Die rasch erfolgende Metabolisierung von 17β-Oestradiol durch die Leber erfordert eine hohe Dosierung, was zu häufigem Auftreten unerwünschter Nebenwirkungen, u.a. Nausea, Thromboembolie etc., führt, siehe hierzu Pharm. Chemie, E. Schröder et al., G. Thieme, Stuttgart 1982, S. 571.

Bei der Langzeitbehandlung mit nicht mit Gestagenen kombinierten Oestrogenen kann ausserdem Endometriumshyperplasie auftreten, die zu einer Erhöhung des Tumorrisikos führt.

Zahlreiche Studien in den USA haben eine 3,3 - 6 mal höhere Inzidenz von Endometriumkarzinomen nach Behandlung mit konjugierten Oestrogenen, Oestradiol oder Oestradiol-Valerat, postuliert. Ebenfalls wird ein Zusammenhang zwischen erhöhtem Oestrogenspiegel und dem Auftreten von Mammakarzinomen vermutet.

Zur Vermeidung solcher Risiken, insbesondere von Endometriums- und Mammakarzinomrisiken, sowie thromboembolischen Komplikationen, wird von P.J. Keller (loc. cit.) die transdermale Behandlung mit Oestrogenen wie 17β-Oestradiol, insbesondere die Verwendung von transdermalen therapeutischen Systemen mit diesem Wirkstoff und eine Ergänzung dieser Behandlung mit Gestagenen vorgeschlagen. Für solche kombinierten Anwendungen stehen bis jetzt aber nur orale Kombinationspräparate zur Verfügung, z.B. wie in der Europäischen Patentanmeldung 136 011 für kontrazeptive Kombinationspräparate vorgeschlagen wird. Daher besteht ein Bedürfnis nach transdermalen Kombinationspräparaten enthaltend Oestrogene und Gestagene, insbesondere transdermalen therapeutischen Systemen mit dieser Wirkstoffkombination.

In der Europäischen Patentanmeldung 196 769 sind transdermale therapeutische Systeme beschrieben, welche kontrazeptive Wirkstoffkombinationen wie Levonorgestrel-Oestradiol enthalten können. Die Wirkstoffe bzw. Wirkstoffkombinationen liegen in mikrodispergierter Form in einer polymeren Matrix vor.

In der DE-A-3,205,258 werden transdermale therapeutische Mebransysteme in Form von Pflastern beschrieben, mit deren Hilfe Oestradiol unter Verwendung des die perkutane Absorption verstärkenden Mittels Aethanol transdermal appliziert werden kann. Der Vorteil solcher Systeme besteht in der geringeren Dosierung von Oestradiol bei Umgehung des Leber-"first pass"-Effektes, so dass bei dieser Applikationsform die Metabolisierung grösserer Wirkstoffmengen unterbleibt.

Für die Applikation von natürlichen und synthetischen Gestagenen haben bisher keine geeigneten transdermalen therapeutischen Systeme zur Verfügung gestanden. So reicht die Durchflussmenge des natürlichen Gestagenderivats Progesteron durch die Haut selbst bei Verwendung des in der DE-A-3,205,258 beschriebenen Vehikels, z.B. geliertem Aethanol, nicht aus, um einen ausreichenden therapeutischen Effekt bei Anwendung transdermaler therapeutischer Systeme vertretbarer Grösse zu erzielen.

Es wurde nun überraschenderweise gefunden, dass die Durchflussmenge des synthetischen Gestagens Norethisteronacetat bei Verwendung von geliertem Niederalkanol wie Aethanol als Vehikel für einen therapeutischen Effekt bei transdermalen Systemem üblicher Grösse (ca. 5 - 25 cm²) ausreicht, so dass die für einen therapeutischen Effekt notwendige Mindestmenge an Gestagen transdermal zugeführt werden kann.

Dieser überraschende Befund lässt sich zur Lösung der dieser Erfindung zugrunde liegenden Aufgabenstellung heranziehen, welche darin besteht, ein geeignetes transdermales therapeutisches System enthaltend 17β-Oestradiol, kombiniert mit Norethisteron-17-acetat, herzustellen.

Die vorliegende Erfindung betrifft ein mehrschichtiges transdermales therapeutisches System bestehend aus:

(1) einer geschlossenen Deckschicht;

(2) einem Reservoir enthaltend eine Wirkstoffkombination aus einem Oestrogenderivat mit einem synthetischen Gestagenderivat und ein die perkutane Absorption verbesserndes Mittel;

(3) einer Klebschicht und

(4) einer abziehbaren Schutzschicht auf der Klebschicht, dadurch gekennzeichnet, dass das Reservoir (2) die Wirkstoffkombination 17beta-Oestradiol mit Norethisteron-17-acetat, ein $C_2$-$C_4$-Alkanol als ein die per-

2

kutane Absorption verbesserndes Mittel und eine permeable Membran enthält.

Das erfindungsgemässe therapeutische System für die transdermale Applikation von 17β-Oestradiol und Norethisteron-17-acetat hat vorzugsweise die Form eines Pflasters mit einer Grundfläche, welche mindestens so gross ist wie die für die Applikation vorgesehene Hautfläche und wie sie für festen Sitz über den gesamten Behandlungszeitraum erforderlich ist. Die Grundfläche muss so gross gewählt werden, dass ausreichende Mengen der aktiven Bestandteile der Wirkstofformulierung (Wirkstoff und die perkutane Absorption verbesserndes Mittel, im folgenden Penetrationsverbesserer) von der Maut aufgenommen werden können. Obwohl sehr grosse Oberflächen der Haut zur Aufnahme des Pflasters theoretisch zur Verfügung stehen, beträgt aus Komfortgründen die maximale Grundfläche des Pflasters ca. 200 cm².

Die geometrische Form des Pflasters ist beliebig, z.B. oval, elliptisch, kreisförmig, rechteckig, gegebenenfalls mit abgerundeten Ecken, oblong oder rechteckig mit ein oder zwei abgerundeten Laschen. Auch andere Formen sind möglich.

Die Deckschicht (1) besteht aus einem Material oder einer Kombination von Materialien, die für die im Reservoir (2) enthaltenen Formulierungsbestandteile undurchlässig sein müssen. Sie dient als Schutz- und als Stützschicht. Zur Herstellung der Deckschicht können Hoch- oder Niederdruckpolymere wie Polyäthylen, Polypropylen, Polyvinylchlorid, Polyäthylenterephthalat oder auch Celluloseacetat oder Vinylacetat-Vinylchlorid-Copolymere und Kombinationen, insbesondere Verbundfolien daraus, verwendet werden. Bevorzugt ist eine undurchlässige, flexible Deckschicht, welche sich der Ausformung der betreffenden Körperpartie anpasst, worauf das Pflaster angebracht ist.

Das Reservoir (2) befindet sich zwischen der Deckschicht (1) und der Klebschicht (3) und enthält wesentliche Formulierungsbestandteile, z.B. die Wirkstoffkombination mit dem Penetrationsverbesserer Aethanol. Ausserdem enthält das Reservoir polymere Materialien zur Bildung einer permeablen Membran.

Das Reservoir (2) kann auch flüssiges polymeres Material enthalten, worin die Formulierungsbestandteile homogen verteilt sind. Solche polymeren Materialien sind z.B. Siliconkautschuk (Silicone), z.B. lineare Organosiloxane, worin jedes Siliciumatom in der Siloxankette durch zwei gleiche oder verschiedene Alkyl-, z.B. Methyl- oder Aethyl-, Aryl-, z.B. Phenyl-, Alkenyl-, z.B. Vinyl- oder Allyl-, Alkylaryl-, z.B. Tolyl- oder Xylyl-, oder Aralkyl-, z.B. Benzylreste, und jedes terminale Siliciumatom durch drei der genannten organischen Reste substituiert ist. Die Herstellung dieser Silicone ist in den U.S. Patentschriften 2,541,137, 2,723,966, 2,863,846, 2,890,188, 2,927,907, 3,002,951 und 3,035,016 beschrieben.

Das Reservoir (2) kann ausser dem flüssigen polymeren Material noch andere Flüssigkeiten wie Glycerin oder Propylenglycol sowie Wasser enthalten und die in der U.S. Patentschrift 4,291,015 beschriebenen Abgabeeigenschaften haben.

Vorzugsweise besteht der Inhalt des Reservoirs (2) ausschliesslich aus dem Penetrationsverbesserer, insbesondere Aethanol, der Wirkstoffkombination 17β-Oestradiol mit Norethisteron-17-acetat und gegebenenfalls weiteren Hilfsstoffen, z.B. Geliermittel.

Das Reservoir (2) ist ausserdem mit einer permeablen Schicht versehen, welche die erforderliche Durchlässigkeit für die Wirkstoffkombination und den Penetrationsverbesserer besitzt. Diese Schicht steuert die Abgabegeschwindigkeit des Penetrationsverbesserers und gegebenenfalls der Wirkstoffkombination aus dem System auf die Haut und wird auch Kontrolloder Steuermembran bezeichnet.

Die für die erfindungsgemässen therapeutischen Systeme verwendbaren Materialien zur Herstellung der permeablen Schicht sind an sich bekannt. Solche Membranmaterialien können homogen (Diffusionsmembranen), oder makrostrukturiert (poröse Membranen) sein. Letztere können als schwammförmiges Gebilde mit einer löchrigen Gerüststruktur aus polymerem Material aufgefasst werden, worin untereinander verbundene Zwischenräume und Poren dispergiert sind. Membranmaterialien, welche die Abgabegeschwindigkeit steuern, können aus isotropem Material mit homogener Struktur oder aus anisotropem Material mit nicht-homogener Struktur bestehen. Solche Materialien sind kommerziell erhältlich und können auf verschiedene Weise hergestellt werden, z.B. wie von R.E. Kesting, Synthetic Polymer Membranes, McGraw Hill, Chapters 4 und 5, 1971, J.D. Ferry, Ultrafiltration Membranes, Chemical Review, Vol. 18, Seite 373, 1984 beschrieben.

Membranmaterialien mit 5 bis 95 Vol% Hohlräumen und einer effektiven Porengrösse von ca. $1,0 \times 10^{-9}$ m bis $1,0 \times 10^{-4}$ m sind besonders geeignet. Vor allem eignen sich Membranmaterialien mit Porengrössen kleiner als ca. $5,0 \times 10^{-9}$ m und molekularer Diffusion. Für optimale Resultate wird auf den Stand der Technik und die bekannten Ausführungsformen mit bekannten Membranmaterialien und bekannter Formgebung verwiesen, welche eine optimale Abgabegeschwindigkeit der Wirkstoffkombination gewährleisten. Insbesondere muss das Membranmaterial gegenüber der Wirkstoffkombination und dem verwendeten Penetrationsverbesserer chemisch resistent sein.

Im folgenden ist eine Aufzählung von geeigneten Membranmaterialien angegeben, welche als nicht erschöpfend aufzufassen ist:

Polycarbonate, z.B. lineare Polyester von Kohlensäurederivaten, welche Carbonatgruppen in der Polymerkette enthalten, und z.B. durch Umsetzung von Dihydroxyaromaten mit Phosgen herstellbar sind. Solche Materialien sind unter dem Warenzeichen Lexan® von General Electric erhältlich;

Polyvinylchloride, z.B. das PVC, welches unter dem Warenzeichen Geon® 121 der Firma Goodrich erhältlich ist;

Polyamide vom Typ Polyhexamethylenadipamid oder solche Polyamide, welche unter dem generischen Namen "Nylon" bekannt sind. Ein besonders geeignetes Material ist unter dem Warenzeichen Nomex® bei DuPont erhältlich;

Acrylsäurecopolymere, z.B. solche, welche unter dem Warenzeichen Dynel® erhältlich sind und zu ca. 60 % aus Polyvinylchlorid und zu 40 % aus Acrylonitril bestehen, sowie Styrol-Acrylsäurecopolymere und ähnliche;

Polysulfone mit Diphenylsulfon-Gruppen in der linearen Kette. Solche Polymere werden von Union Carbide unter der Bezeichnung P-1700 angeboten;

Halogenierte Polymere wie Polyvinylidenfluoride, welche z.B. unter dem Warenzeichen Kynar® von Pennwalt vertrieben werden; Polyvinylfluoride, welche unter dem Warenzeichen Tedlar® bei DuPont erhältlich sind, sowie Polyfluorohalocarbon erhältlich unter dem Warenzeichen Aclar® bei Allied Chemical;

Polychloräther, welche unter dem Warenzeichen Penton® von Hercules vertrieben werden, sowie andere ähnliche thermoplastische Polymere;

Acetalpolymere wie die Polyformaldehyd-Polymere, welche von DuPont unter dem Warenzeichen Delrin® vertrieben werden u.ä.

Acrylsäureresinate wie Polymethylmethacrylat, Poly-n-butyl-methacrylat u.ä.

Polyäthylen und Copolymere des Aethylens z.B. mit Vinylacetat oder Acrylaten.

Andere Polymere wie Polyurethane, Polyimide, Polybenzimidazole, Polyvinylacetat, aromatische und aliphatische Polyäther, Celluloseester, z.B. Cellulosetriacetat, Cellulose, Colledion® (Cellulosenitrat mit 11 % Stickstoff), Epoxyresinate, Polyolefine, z.B. Polyethylen-polypropylen, poröses Gummi, Polyvinylpolypyrrolidon, quervernetzter Polyvinylalkohol, Copolymerisate aus Vinylpyrrolidon und Vinylalkoholen, Polyelektrolytstrukturen, welche aus zwei ionisch assoziierten Polymeren bestehen, wie in den U.S. Patentschriften 3,549,016 und 3,546,142 beschrieben, Polystyrolderivate wie Polystyrolnatriumsulfonate oder Polyvinylbenzyltrimethylammoniumchloride, Polyhydroxyäthylmethylacrylate, Polyisobutylvinyläther und ähnliche Polymere können ebenfalls verwendet werden. Weitere Copolymere, welche durch Copolymerisation von verschiedenen Mengen der den genannten Polymeren zugrundeliegenden Monomeren erhältlich sind, sind ebenfalls zur Herstellung des die Abgabe-geschwindigkeit des Penetrationsverbesserers bestimmenden Membranmaterials verwendbar.

Die permeable Membran kann unterschiedlich angeordnet sein: Die Wirkstoffkombination befindet sich zwischen der Deckschicht (1) und der Membranschicht. In dieser Anordnung wird von der Deckschicht und der Membran ein Volumen gebildet, welches gegebenenfalls durch mehrere Kammern unterteilt sein kann. Die Deckschicht (1) und die Membranschicht sind bei bestimmten Ausführungsformen am Rand selbst miteinander verbunden, z.B. verschweisst oder verklebt. In diesen Ausführungsformen sind die Wirkstoffkombination und der Penetrationsverbesserer im selben Reservoir enthalten. Diese Ausführungsformen sind bevorzugt, wenn die Formulierungsbestandteile flüssig oder halbfest sind.

Man kann auch gemäss der in der Deutschen Offenlegungsschrift 3,205,258 beschriebenen Ausführungsform das von der Deckschicht (1) und der Membran gebildete Volumen nur mit dem Penetrationsverbesserer Aethanol, und gegebenenfalls mit einem Geliermittel wie Gelatine füllen und auf die andere Seite der Membran die Wirkstoffkombination auftragen. Die Membran würde hierbeinur die Diffusionsgeschwindigkeit des Penetrationsverbesserers steuern. Die Wirkstoffkombination kann in einer getrennten Schicht zwischen Membran und Klebschicht (3) und gegebenenfalls oder ausschliesslich in der Klebschicht (3) vorhanden sein.

Das Reservoir (2) kann ausserdem in mehrere Kammern unterteilt sein. Diese Unterteilung eignet sich für flüssige Wirkstoffformulierungen und verhindert, dass diese bei Bildung von Hohlräumen oder Falten infolge unebener Lagerung des Pflasters absinken und sich am tiefsten Punkt des Systems anreichern. Besonders vorteilhaft ist eine Unterteilung, wenn die Reservoirschicht eine Fläche von mehr als 30 cm² einnimmt. Die Einteilung der Kammern ist beliebig. So sind z.B. die radiale Anordnung der Stege vom Mittelpunkt des Pflasters, vertikale, horizontale Abgrenzungen, schräge Linien etc. möglich.

Die Unterteilung der Kammern, insbesondere durch Stege oder Siegelnähte, kann durch Verschweissen unter Wärme vorgenommen werden. Dabei wird das Material der Deckschicht (1) mit dem Material der Membranschicht verschweisst.

Für die Klebschicht (3) eignen sich die in der Dermatologie verwendbaren Klebematerialien. Geeignete Klebematerialien sind beispielsweise klebrige Formulierungen von Acrylsäure- oder Methacrylsäureharzen, z.B. Polymere von Acrylsäure oder Methacrylsäure verestert mit Alkoholen wie n-Butanol, n-Pentanol, Isopentanol, 2-Methylbutanol, 1-Methylbutanol, 1-, 2- oder 3-Methylpentanol, 2-Aethylbutanol, Isooctanol, n-Decanol oder n-Dodecanol, oder Copolymerisate dieser Acrylsäure- oder Methacrylsäureester mit Aethylengruppen-haltigen Monomeren wie Acrylsäure selbst, Methacrylsäure, Acrylamid, Methacrylamid, N-Alkoxymethacrylamid, N-Alkoxymethylmethacrylamid, N-tert-Butylamid, Itaconsäure, Vinylacetat, N-verzweigtes Alkylmaleinsäureamid, worin die verzweigte Alkylgruppe 10-24 C-Atome hat, Glycoldiacrylate oder Mischungen davon, natürlicher oder synthetischer Kautschuk wie Styrolbutadien, Butyläther, Neopren, Polyisobutylen, Polybutadien und Polyisopren, Polyvinylacetat, Harnstoff-Formaldehyd-Resinate, Resorcin-Formaldehyd-Resinate, Cellulosederivate wie Aethylcellulose, Methylcellulose, Nitrocellulose, Celluloseacetatbutyrat und Carboxymethylcellulose sowie natürliche Kleber wie Guar, Acacia, Pektin, Stärke, Dextrin, Albumin, Gelatine, Casein etc. Die genannten Klebstoffe können noch mit Verdickern und Stabilisatoren versetzt werden.

Die Klebschicht (3) kann teilweise oder vollständig auf der Membran aufgetragen sein. Bei vollständiger Bedeckung der Membran mit Klebschicht (3) kann diese neben ihrer eigentlichen Funktion als Haftmittel auf der Haut ausserdem als permeable Membran wirken. Die gewünschten Membraneigenschaften, z.B. Steuerung der Diffusionsgeschwindigkeit des Penetrationsverbesserers, lassen sich durch Variation der Stärke und Zusammensetzung der Klebschicht erzielen. Die Klebschicht (3) kann ausserdem die gesamte oder vorzugsweise eine anteilige Menge der Wirkstoffkombination enthalten. Mit der in der Klebschicht enthaltenen Wirkstoffmenge lässt sich insbesondere eine anfängliche stossweise Zufuhr erzielen, bevor die vom therapeutischen System gesteuerte kontinuierliche Zufuhr auf dem gewünschten therapeutischen Niveau einsetzt.

Die Membran kann ausserdem teilweise und/oder diskontinuierlich mit der Klebschicht (3) bedeckt sein. Dabei ist eine randförmige Bedeckung möglich, z.B. ringförmige umlaufende Bedeckung. Die Membran kann auch musterförmig bedeckt sein, z.B. rautenförmig. Die Membran kann am äusseren Rand durch ein kontinuierliches Band von Klebematerial, z.B ringförmig, und auf der Innenfläche mit diskontinuierlichen Bändern, z.B. rautenförmig, bedeckt sein.

Die Schutzschicht (4) wird vor der Applikation entfernt. Sie besteht aus Materialien, die für die Bestandteile der Reservoirschicht (2) undurchlässig sind. Dabei lassen sich dieselben Materialien, die zur Herstellung der Deckschicht (1) dienen können, sowie Metallfolien, z.B. dünne Aluminiumfolie, verwenden. Organische Polymere werden durch geeignete Oberflächenbehandlung, z.B. Siliconbehandlung, von der Klebschicht (3) abziehbar gemacht.

Die im erfindungsgemässen transdermalen therapeutischen System, insbesondere im Reservoir (2), enthaltenen Formulierungsbestandteile enthalten als Hilfsstoff ein $C_2$-$C_4$-Alkanol als ein die perkutane Absorption verstärkendes Mittel (Penetrationsverbesserer), welches die Durchflussgeschwindigkeit (flux) der Wirkstoffkombination durch die Haut erhöht, so dass eine grössere Menge an Wirkstoffen bezogen auf die Applikationsfläche und Zeiteinheit von der Haut absorbiert wird. Der Penetrationsverbesserer kann ausserdem den Durchgang der Wirkstoffe durch die permeable Membranschicht in Membransystemen beschleunigen. Insbesondere wird bei Verwendung von Aethanol die zur Einhaltung des therapeutischen Niveaus notwendige Dosismenge an Wirkstoffen pro Zeiteinheit durch die Haut appliziert.

Als Penetrationsverbesserer sind $C_2$-$C_4$-Alkanole, z.B. Isopropanol oder Isobutanol, sowie vor allem Aethanol, geeignet.

Die zur Erzielung eines therapeutischen Effekts im therapeutischen System enthaltene Wirkstoffmenge ist von vielen Faktoren abhängig: u.a. von der erforderlichen Mindestdosismenge, der Permeabilität des die Durchtrittsgeschwindigkeit bestimmenden Membranmaterials und der Klebschicht sowie der Zeitdauer, in der das Pflaster auf der Haut oder den Schleimhäuten befestigt ist. Da sich die Wirkstoffabgabe über einen längeren Zeitraum als einen Tag erstrecken soll, besteht eigentlich keine Obergrenze hinsichtlich der maximalen im Pflaster enthaltenen Wirkstoffmengen. Die Mindestwirkstoffmenge wird vom Erfordernis festgelegt, dass ausreichende Wirkstoffmengen im Pflaster enthalten sein müssen, um die erwünschte Abgaberate über den vorgesehenen Zeitraum aufrechtzuerhalten.

Die im Reservoir (2) enthaltene Wirkstoffkombination besteht aus 17β-Oestradiol und Norethisteron-17-acetat.

Die im erfindungsgemässen therapeutischen System enthaltene Wirkstoffkombination hat den Vorteil, dass sich damit Oestrogene und Gestagene kombiniert dermal applizieren lassen. Diese Applikationsform ist insbesondere wegen der Vereinfachung der Applikationsweise im Vergleich zur Kombination von transdermaler und oraler Applikation sowie wegen der Vermeidung des Leber-"first pass"-Effekts vorteilhaft, so dass niedrigere Dosismengen als bei der bisher üblichen peroralen Applikation zur Anwendung gelangen können.

Im erfindungsgemässen therapeutischen System sind z.B. ca. 0,2 - 20 mg 17β-Oestradiol und ca. 0,5 - 60 mg Norethisteron-17-acetat enthalten. In bevorzugten Darreichungsformen sind ca. 2,0 - 5,0 mg 17 β-Oestradiol und ca. 5,0 - 30 mg Norethisteron-17-acetat enthalten. Diese Mengen reichen aus, um selbst bei mehrtägigem Tragen des Pflasters die Abgabe und Absorption von täglichen therapeutischen Mindestmengen von ca. 0,05 mg 17β-Oestradiol und ca. 0,2 mg Norethisteron-17-acetat zu gewährleisten.

Der Wirkstoffkombination können Hilfsstoffe zugesetzt sein. Geeignet sind Hilfsstoffe wie Wasser, isotonische wässrige Kochsalzlösung, Dextrose in Wasser oder Kochsalzlösung, flüssige Glyceryltriester mit niedermolekularen Fettsäuren, Niederalkanole, natürliche Oele wie Maisöl, Erdnussöl, Sesamöl, Castoröl sowie deren Kondensationsprodukte mit Aethlyenoxid und ähnliche, Kohlenwasserstoffe wie Mineralöl pharmazeutischer Qualität, Silicone, Emulgatoren wie Mono-oder Diglyceride von Fettsäuren, Phosphatidsäurederivate wie Lecithin oder Kephalin, Polyalkylenglycole wie Polyäthylenglycol, wässrige Phasen versetzt mit einem Quellmittel wie Natriumcarboxymethylcellulose, Natriumalginat, Polyvinylpolypyrrolidon etc., denen noch Dispersionsmittel oder Emulgatoren wie Lecithin zugesetzt sein können, Polyoxyäthylen und ähnliche. Die Hilfsstoffe können ferner Zusätze wie Konservierungsmittel, Stabilisatoren, Benetzungsmittel, Emulgatoren etc. enthalten.

Den $C_2$-$C_4$-Alkanolen wie Aethanol als Penetrationsverbesserer werden als Hilfsstoffe vorzugsweise Geliermittel wie Gelatine oder Quellmittel wie Celluloseäther, z.B. Hydroxypropylcellulose der Wirkstoffformulierung zugesetzt.

Die erfindungsgemässen transdermalen therapeutischen Systeme werden auf an sich bekannte Weise hergestellt, z.B. indem man die Klebschicht (3) auf einer Grundlage (abziehbare Schutzschicht (4)), z.B. Folie oder Film, aufträgt. Auf die Grundlage können auch die Bestandteile des Wirkstoffreservoirs (2), z.B. Membranschicht und Wirkstoffkombination, aufgetragen und darauf die undurchlässige Deckschicht (1) gelegt werden. Anschliessend wird das Pflaster aus der Vorlage ausgestanzt. Gegebenenfalls wird das Reservoir mit zusätzlichem Klebstoff mit der Deckschicht verbunden. Ebenso kann das Reservoir mit der Membranschicht oder mit der Klebschicht heiss verschweisst werden. Bei flüssigkeitsgefüllten Systemen wird die Membranschicht auf die Klebschicht (3) aufgetragen und auf die Membran die Wirkstoffformulierung gebracht.

Die Herstellungsverfahren sind in der U.S. Patentschrift Nr. 3,797,494 beschrieben, vorzugsweise in der DE-A-26 04 718 und DE-A-32 05 258 bzw. in den U.S. Patentschriften 4,031,894 und 4,262,003 oder in der Publikation von H. Asche in Schweiz. Rundschau Med. (Praxis) 74, Nr. 11, 257-260 (1985), wobei die erfindungsgemässe Anwendung nicht auf die in diesen Publikationen beschriebenen transdermalen therapeutischen Systeme beschränkt ist. Das bevorzugte transdermale therapeutische System gemäss DE-A 32 05 258 ist ein therapeutisches System in Form einer pflasterartigen Auflage, das die Wirkstoffkombination transdermal unter Vermeidung von Nebenwirkungen in einer Menge von 0,3 bis 15 µg/h freisetzt und durch die Haut abgibt, so dass der Wirkstoffgehalt im Plasma annähernd konstant aufrechterhalten bleibt.

Das erfindungsgemässe transdermale therapeutische System eignet sich zur Applikation der Wirkstoffkombination aus 17β-Oestradiol und Norethisteron-17-acetat bei der Behandlung von sämtlichen Krankheitszuständen, die von Oestradiolmangel verursacht werden, z.B. Osteoporose, Kopfschmerzen, Uebelkeit, Depressionen, Hitzewallungen etc., insbesondere Krankheitsbildern, die unter dem Oberbegriff "Klimakterisches Syndrom" zusammengefasst werden.

Das erfindungsgemässe transdermale therapeutische System eignet sich ausserdem zur zyklisch intermittierenden Behandlung des klimakterischen Syndroms, indem man z.B. zwei oder vorzugsweise drei Wochen lang transdermale therapeutische Systeme mit 17β-Oestradiol als Wirkstoff, z.B. das System Estraderm®-TTS (Ciba-Geigy), appliziert und anschliessend das erfindungsgemässe transdermale therapeutische System mit der Wirkstoffkombination aus 17β-Oestradiol und Norethisteron-17-acetat zwei oder vorzugsweise eine Woche lang anwendet. Diese Behandlung wird anschliessend zyklisch wiederholt.

Die folgenden Beispiele illustrieren die Erfindung ohne diese zu beschränken:

Beispiel 1:

Ein transdermales therapeutisches System in Form eines Pflasters zur kombinierten Verabreichung von 17β-Oestradiol und Norethisteron-17-acetat wird folgendermassen hergestellt:

Eine Lösung von Oestradiol und Norethisteronacetat in 95%-igem Ethanol wird durch Vermischen von 0,0430 Gew.-Teilen Norethisteron17-acetat und 0,0134 Gew.-Teilen Oestradiol in 1,000 Gew.-Teilen 95%-igem Aethanol hergestellt. Die Lösung wird durch Zugabe von 0,0188 Teilen Hydroxypropylcellulose (MG 1'000'000, Klucel®) unter Rühren geliert.

Anschliessend wird ein Kontaktklebemittel hergestellt und zwar durch Vermischen von Polyisobutylen (MG 1 200 000), Polyisobutylen (MG 35 000) und einem leichten Mineralöl im Gewichtsverhältnis 1,0:1,27:2,218. Man löst diese Mischung in Leichtbenzin, so dass eine 35%-ige Lösung entsteht, giesst eine ca. 50 µm starke Schicht dieser Kontaktkleberlösung auf eine 75 µm starke Folie aus Silicon-behandeltem Polyethylenterephthalat (Release liner) und trocknet bei Raumtemperatur. Auf die Kontaktklebemittelschicht der erhaltenen zweischichtigen Grundlage wird die Kontrollmembran in Form eines 50 µm starken Films aus Ethylenvinylacetat-Copolymer (EVA, 9 % Vinylacetat) laminiert. Das erhaltene aus drei Schichten bestehende Laminat wird in Stücke von 15 x 11 $cm^3$ geschnitten. Vier Anteile von je 400 mg des gelierten Oestradiol-NorethisteronacetatEthanol-Gemisches werden in gleichmässigem Abstand auf die EVA-Copolymerseite jedes Stückes aufgebracht und ein 63,5 μm dicker Film für die Rückseite aus gegebenenfalls aluminisiertem Polyethylenterephthalat, mit einem mit EVA in der Witze versiegelbarem Ueberzug über die Gele gelegt. Die Rückseitenfolie wird am äusseren Rand jedes Stückes bei 130°C und 27 kg mit dem EVA-Copolymer versiegelt. Die fertigen Pflaster werden aus dem Laminat mit einer Stanze mit einem Durchmesser von 4 cm ausgestanzt.

Beispiel 2:

a) In-vitro Tests werden durchgeführt, um den Austritt von Oestradiol und Norethisteronacetat aus den oben beschriebenen Pflastern zu bestimmen. Dabei wird das grundlegende Verfahren von Chandrasekaran, et al, Am. Inst. Chem. Eng. J., 22, 828 (1976) angewandt. Die Norethisteronacetat- und die Oestradiol-Konzentration in der Rezeptor-Flüssigkeit werden chromatographisch (HPLC) bestimmt.

b) Resultate:

Norethisteronacetat:

| Abgabegeschwindigkeit $[\mu g / cm^2\ h]$ | | insgesamt abgegebene Menge $[\mu g / cm^2]$ |
|---|---|---|
| 0 - 24 h | 24 - 48 h | 0 - 96 h |
| 2,9 | 3,7 | 182 |

Oestradiol:

| Abgabegeschwindigkeit $[\mu g / cm^2\ h]$ | | insgesamt abgegebene Menge $[\mu g / cm^2]$ |
|---|---|---|
| 0 - 24 h | 24 - 48 h | 0 - 96 h |
| 0,17 | 0,23 | 12 |

Beispiel 3:

Durchflussgeschwindigkeiten von Norethisteronacetat verglichen mit anderen synthetischen Progesteronderivaten werden in-vitro wie folgt gemessen:
In einer Diffusionszelle nach T. J. Franz, J. Invest. Dermatol. 64, 190 - 195 (1975) wird im Donorkompartiment gesättigte äthanolische Lösung der betreffenden Gestagene oder das transdermale therapeutische System gemäss Beispiel 1 in direkten Kontakt mit der zwischen Donor- und Akzeptorkompartiment eingespannten Schweinehaut (ca. 2 cm²) gebracht. Aus dem Akzeptorkompartiment wird nach 24 Stunden ein Aliquot der wässrigen Akzeptorlösung auf den Gehalt des betreffenden Progesteronderivats untersucht und daraus die Durchflussgeschwindigkeiten in μg/cm² x h berechnet.

EP 0 285 563 B1

| Testverbindung | Therapeutisch wirksame Tagesdosis [mg] | | Hautpermeation-in vitro | | Notwendige Systemfläche [1) [cm²] |
|---|---|---|---|---|---|
| | oral | transdermal[1]) | alkoholische Lösung [µg/cm²·h] | System gemäss Beispiel 1 [µg/cm²·h] | |
| Norethisteron | 0,5-5 | 0,2 | 0,024 | | > 350 |
| Medroxyprogesteronacetat | 2,5-10 | 2 | 0,019 | | > 4380 |
| d-Norgestrel | 0,07-0,15 | 0,05 | 0,028 | | 74 |
| Norethisteronacetat | 0,5-5 | 2 | 1,47 | 0,39 | 21 |

[1]) Schätzwerte

**Patentansprüche**

**Patentansprüche für folgende Vertragsstaaten: AT, BE, CH, DE, FR, GB, IT, LI, LU, NL, SE**

1. Mehrschichtiges transdermales therapeutisches System bestehend aus:
(1) einer geschlossenen Deckschicht;
(2) einem Reservoir enthaltend eine Wirkstoffkombination aus einem Oestrogenderivat mit einem synthetischen Gestagenderivat und ein die perkutane Absorption verbesserndes Mittel;
(3) einer Klebschicht und
(4) einer abziehbaren Schutzschicht auf der Klebschicht, dadurch gekennzeichnet, dass das Reservoir (2) die Wirkstoffkombination 17beta-Oestradiol mit Norethisteron-17-acetat, ein $C_2$-$C_4$-Alkanol als ein die perkutane Absorption verbesserndes Mittel und eine permeable Membran enthält.

2. Mehrschichtiges transdermales therapeutisches System gemäss Anspruch 1, dadurch gekennzeichnet, dass das Reservoir (2) Aethanol als ein die perkutane Absorption verbesserndes Mittel enthält.

3. Verfahren zur Herstellung von therapeutischen Systemen gemäss Anspruch 1 für die transdermale Applikation von Oestrogenen und Gestagenen, dadurch gekennzeichnet, dass man auf der abziehbaren Schutzschicht (4) nacheinander die Bestandteile des therapeutischen Systems aufträgt und gegebenenfalls miteinander verschweisst.

4. Therapeutisches System gemäss Anspruch 1 zur Anwendung bei der Behandlung des klimakterischen Syndroms.

**Patentansprüche für folgende Vertragsstaaten: ES, GR**

1. Verfahren zur Herstellung eines mehrschichtigen transdermalen therapeutischen Systems bestehend aus:
(1) einer geschlossenen Deckschicht;
(2) einem Reservoir enthaltend eine Wirkstoffkombination aus einem Oestrogenderivat mit einem synthetischen Gestagenderivat und ein die perkutane Absorption verbesserndes Mittel;
(3) einer Klebschicht und
(4) einer abziehbaren Schutzschicht auf der Klebschicht, dadurch gekennzeichnet, dass man das Reservoir (2) mit der Wirkstoffkombination 17beta-Oestradiol und Norethisteron-17-acetat, mit einem $C_2$-$C_4$-Alkanol als einem die perkutane Absorption verbessernden Mittel und einer permeablen Membran versieht.

2. Verfahren zur Herstellung eines mehrschichtigen transdermalen therapeutischen Systems gemäss Anspruch 1, dadurch gekennzeichnet, dass man das Reservoir (2) mit Aethanol als einem die perkutane Absorption verbessernden Mittel versieht.

3. Verfahren zur Herstellung von therapeutischen Systemen gemäss Anspruch 1 für die transdermale Applikation von Oestrogenen und Gestagenen, dadurch gekennzeichnet, dass man auf der abziehbaren Schutzschicht (4) nacheinander die Bestandteile des therapeutischen Systems aufträgt und gegebenenfalls miteinander verschweisst.

**Claims**

**Claims for the following Contracting States: AT, BE, CH, DE, FR, GB, LI, LU, NL, SE**

1. A multi-layered transdermal therapeutic system consisting of:
(1) a closed outer layer;
(2) a reservoir comprising an active ingredient combination of an oestrogen derivative with a synthetic gestagen derivative, and an agent that enhances percutaneous absorption;
(3) an adhesive layer and
(4) a peel-off protective layer on the adhesive layer, characterised in that the reservoir (2) comprises the active ingredient combination 17β-oestradiol with norethisterone-17-acetate, a $C_2$-$C_4$alkanol as an agent that enhances percutaneous absorption, and a permeable membrane.

2. A multi-layered transdermal therapeutic system according to claim 1, characterised in that the reservoir (2) comprises ethanol as an agent that enhances percutaneous absorption.

3. A process for the manufacture of a therapeutic system according to claim 1 for the transdermal administration of oestrogens and gestagens, characterised in that the components of the therapeutic system are applied to the peel-off protective layer (4) one after another and, if desired, are welded together.

4. A therapeutic system according to claim 1 for use in the treatment of the climacteric syndrome.

**Claims for the following Contracting States: ES, GR**

1. A process for the manufacture of a multi-layered transdermal therapeutic system consisting of:
(1) a closed outer layer;
(2) a reservoir comprising an active ingredient combination of an oestrogen derivative with a synthetic gestagen derivative, and an agent that enhances percutaneous absorption;
(3) an adhesive layer and
(4) a peel-off protective layer on the adhesive layer, characterised in that the reservoir (2) is provided with the active ingredient combination $17\beta$-oestradiol with norethisterone-17-acetate, with a $C_2$-$C_4$ alkanol as an agent that enhances percutaneous absorption, and a permeable membrane.

2. A process for the manufacture of a multi-layered transdermal therapeutic system according to claim 1, characterised in that the reservoir (2) is provided with ethanol as an agent that enhances percutaneous absorption.

3. A process for the manufacture of a therapeutic system according to claim 1 for the transdermal administration of oestrogens and gestagens, characterised in that the components of the therapeutic system are applied to the peel-off protective layer (4) one after another and, if desired, are welded together.

**Revendications**

**Revendications pour les Etats contractants suivants: AT, BE, CH, DE, FR, GB, IT, LI, LU, NL, SE**

1. Système thérapeutique transdermique à couches multiples consistant en
(1) une couche de couverture fermée ;
(2) un réservoir contenant une combinaison de substances actives consistant en un dérivé d'oestrogène et un dérivé synthétique de gestagène et un agent améliorant l'absorption percutanée ;
(3) une couche adhésive, et
(4) une couche de protection éliminable sur la couche adhésive, caractérisé en ce que le réservoir (2) contient la combinaison de substances actives 17 bêta-oestradiol et 17-acétate de noréthistérone, un alcanol en C 2-C 4 en tant qu'agent améliorant l'absorption percutanée, et une membrane perméable.

2. Système thérapeutique transdermique à couches multiples selon revendication 1, caractérisé en ce que le réservoir (2) contient de l'éthanol en tant qu'agent améliorant l'absorption percutanée.

3. Procédé de préparation des systèmes thérapeutiques selon revendication 1 pour l'administration transdermique d'oestrogènes et de gestagènes, caractérisé en ce que, sur la couche de protection éliminable (4), on applique successivement les composants du système thérapeutique et le cas échéant on les soude entre eux.

4. Système thérapeutique selon revendication 1 pour l'utilisation pour le traitement du syndrome climatérique.

**Revendications pour les Etats contractants: ES, GR**

1. Procédé de préparation d'un système thérapeutique transdermique à couches multiples consistant en :
(1) une couche de couverture fermée ;
(2) un réservoir contenant une combinaison de substances actives consistant en un dérivé d'oestrogène et un dérivé synthétique de gestagène et un agent améliorant l'absorption percutanée ;
(3) une couche adhésive, et
(4) une couche protectrice éliminable sur la couche adhésive, caractérisé en ce que l'on introduit dans le réservoir (2) la combinaison de substances actives 17 bêta-oestradiol et 17-acétate de noréthistérone, un alcanol en C 2-C 4 servant d'agent améliorant l'absorption percutanée et une membrane perméable.

2. Procédé de préparation d'un système thérapeutique transdermique à couches multiples selon revendication 1, caractérisé en ce que l'on introduit dans le réservoir (2) de l'éthanol en tant qu'agent améliorant l'absorption percutanée.

3. Procédé de préparation de système thérapeutique selon revendication 1 pour l'administration transdermique d'oestrogènes et de gestagènes, caractérisé en ce que, sur la couche de protection éliminable (4), on applique successivement les composants du système théapeutique et le cas échéant on les soude entre eux.